# EUROPEAN PATENT SPECIFICATION

(11) **EP 3 972 677 B1**
(45) Date of publication and mention of the grant of the patent: **26.02.2025**
(21) Application number: 20734246.0
(22) Date of filing: 23.04.2020
(51) Int. Cl.: A61M 16/04

(54) **TRACHEOSTOMY TUBE ASSEMBLY AND METHOD OF FORMING A PAEDIATRIC TRACHEOSTOMY TUBE ASSEMBLY**
TRACHEOSTOMIEROHRANORDNUNG UND VERFAHREN ZUR HERSTELLUNG EINER PÄDIATRISCHEN TRACHEOSTOMIEROHRANORDNUNG
ENSEMBLE TUBE DE TRACHÉOTOMIE ET PROCÉDÉ DE FORMATION D'UN ENSEMBLE TUBE DE TRACHÉOTOMIE PÉDIATRIQUE

(30) Priority: 20.05.2019 GB 201907094
(43) Date of publication of application: 30.03.2022
(73) Proprietor: ICU Medical International Limited, Lower Pemberton Ashford, Kent TN25 4BF (GB)
(72) Inventor: LACY, Christopher, Allen, Arden Hills, MN 55112 (US)
(74) Representative: Flint, Jonathan McNeill
(86) International application number: PCT/GB2020/000056
(87) International publication number: WO 2020/234554

(56) References cited:
- WO-A1-93/24170
- DE-A1- 3 433 785
- US-A- 1 268 470
- US-A1- 2002 157 665
- US-A1- 2014 190 487
- US-A1- 2014 271 255
- US-A1- 2014 360 508
- US-A1- 2015 080 793
- US-A1- 2015 290 410
- US-A1- 2019 009 042
- US-B2- 6 776 157
- US-B2- 8 863 746

## Description

This invention relates to tracheostomy tube assemblies of the kind including a tracheostomy tube having a shaft with an inflatable sealing cuff towards its patient end and an inflation line communicating with the sealing cuff and having an inflation indicator towards its machine end.

Tracheal tubes are used to enable ventilation, respiration or spontaneous breathing of a patient. Endotracheal tubes are inserted via the mouth or nose so that one end locates in the trachea and the other end locates outside the patient. Tracheostomy tubes are inserted into the trachea via a surgically-formed opening in the neck. Tracheostomy tubes can be inserted by different techniques, such as the surgical cut-down procedure carried out in an operating theatre or a cricothyroidotomy procedure, which may be carried out in emergency situations.

Tracheostomy tubes are generally used for more long-term ventilation or where it is not possible to insert an airway through the mouth or nose. The patient is often conscious while breathing through a tracheostomy tube, which may be open to atmosphere or connected by tubing to some form of ventilator. The tube is secured in position by means of a flange fixed with the machine end of the tube and positioned to extend outwardly on opposite sides of the tube.

Tracheal tubes often have a sealing cuff on their outside close to the patient end. This usually takes the form of a cuff that is inflated by a gas or liquid so that, when inflated, it forms a seal with the tracheal wall, confining gas flow along the bore of the tube and preventing flow around its outside. The cuff is usually inflated and deflated by means of an inflation line in the form of a small-bore tube opening into the cuff at its patient end and extending along a channel along the outside of the shaft of the tube. The machine end of the inflation line extends freely beyond the channel and is terminated by a combined inflation indicator, connector and valve. Alternatively, the tracheal tube may have an inflation lumen extending within the wall of the shaft of the tube and joined towards the machine end of the shaft with a separate small-bore inflation tube. The nose of a syringe or the like is inserted in the connector, opening the valve so that inflation gas or liquid can be supplied to or from the sealing cuff via the inflation line. The inflation indicator usually takes the form of a small flexible envelope or balloon connected at one end with the connector. The inflation line extends and opens into the interior of the inflation indicator so that the indicator is inflated by pressure in the sealing cuff and inflation line. If the pressure in the sealing cuff should fall this would be indicated by a corresponding deflation of the inflation indicator.

A problem occurs when tracheostomy tubes with such indicators are used with children. The soft nature of the inflation indicator and the proximity of the inflation indicator to the child's mouth means that the indicator can be easily chewed or bitten. This is a problem because, if the indicator or inflation line should be damaged, it may permit the inflation gas or liquid to escape thereby allowing the sealing cuff to deflate. This could allow ventilation gas to pass around the outside of the tracheostomy tube and prevent correct ventilation of the patient. The child chewing the inflation line and indicator may also ingest a part of the indicator.

Tracheal tubes with inflatable sealing cuffs and inflation indicators having a protective enclosure, according to the preamble of claim 1, are described in US2014/190487,

US2002/157665, US2014/271255, WO93/24170, US2015/290410, US2019/009042, and US2015/080793. DE3433785 A1 discloses an endotracheal tube with an inflatable sealing cuff and an inflation indicator having a protective enclosure.

It is an object of the present invention to provide an alternative tracheostomy tube assembly.

According to the present invention there is provided a tracheostomy tube assembly according to claim 1, and a method according to claim 6. The dependent claims define embodiments of the invention.

A paediatric tracheostomy tube assembly according to the invention as claimed, with an inflation indicator and protector, will now be described, by way of example, with reference to the accompanying drawings in which:
- Figure 1: is a perspective view of the tracheostomy tube assembly;
- Figure 2: is an enlarged perspective view of the inflation indicator from one end;
- Figure 3: is an enlarged perspective view of the inflation indicator from the opposite end;
- Figure 4: shows the parts of the inflation indicator before assembly;
- Figure 5: is a perspective view of a modified embodiment of the protector from its patient end; and
- Figure 6: is a perspective view of the modified embodiment of the protector from its machine end.

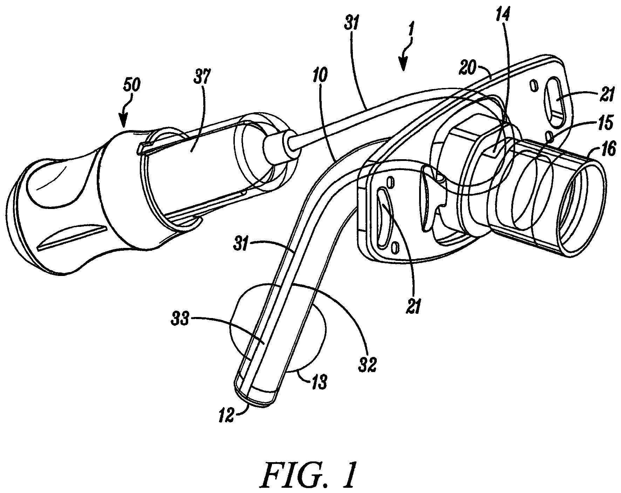

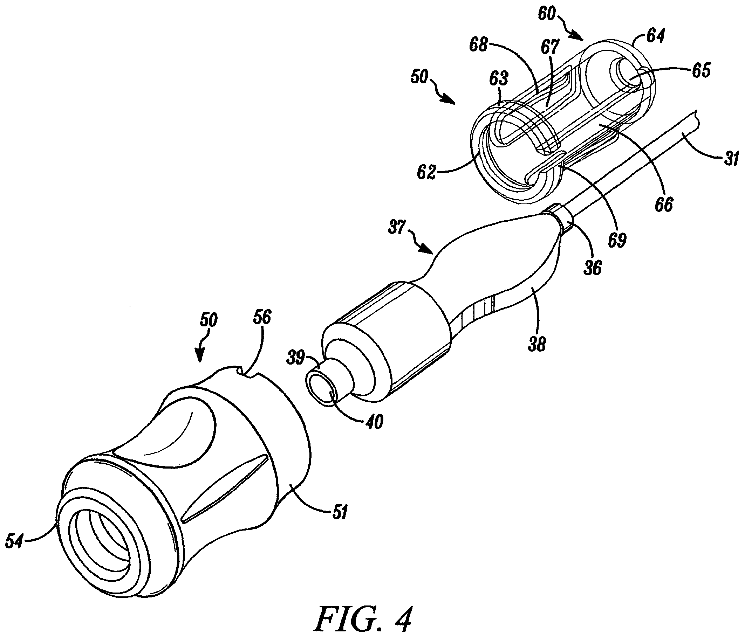

With reference first to Figure 1 to 4, the assembly includes a tracheostomy tube 1 of a paediatric size having a curved shaft 10 of circular section and with an inner diameter of around 6mm. The shaft 10 has a patient end 12 adapted to be located within the trachea of the patient and has a conventional sealing cuff 13 towards its patient end. The cuff 13 shown is of a high pressure kind made of an elastic material that lies close to the shaft 10 when deflated and is stretched outwardly when inflated. The shaft 10 and cuff 13 are both moulded of a silicone material. It will be appreciated that the tube could be of a different sizes, shapes and materials according to the application.

The machine end 14 of the shaft 10 is adapted, during use, to be located externally of the tracheostomy opening formed in the patient's neck. The machine end 14 of the shaft 10 is bonded into a hub or connector 15 having a conventional 15mm male tapered outer surface 16. The connector 15 is adapted to make a removable push fit in a conventional 15mm female connector (not shown) at one end of a breathing tube extending to a ventilator or anaesthetic machine. Alternatively, the machine end of the tube 1 could be left open to atmosphere when the patient is breathing spontaneously. The tracheostomy tube 1 also includes a radially-extending support flange 20 adapted to lie against the skin surface of the neck on either side of the tracheostomy stoma. The flange 20 is moulded integrally as one part with the shaft 10 at its machine end 14 or may be formed separately. The flange 20 has openings 21 at opposite ends for attachment to a neck strap (not shown) used to support the tube with the patient's neck.

The shaft 10 also includes an inflation line 31 in the form of a small-bore tube secured in a channel 32 extending along the outside of one side of the shaft. The inflation line 31 has an opening 33 towards its patient end located approximately midway along the length of the sealing cuff 13. The machine end of the inflation line 31 extends through the flange 20 and continues unattached with the shaft 10 being joined with and extending into one end 36 a conventional inflation indicator 37. The inflation indicator 37 includes a flat, flexible envelope or balloon 38 into which the inflation line 31 opens so that the balloon is inflated or deflated according to the inflation state of the sealing cuff 13. The opposite end of the inflation indicator balloon 38 is bonded to one end of the outside of a rigid connector sleeve 39 containing a valve (not visible in the drawing) and being open at its machine end 40 to receive a male coupling such as the nose of an inflation syringe.

As so far described the tracheostomy tube assembly is conventional. The assembly differs from conventional tracheostomy tubes by the addition of a protector enclosure 50 adapted to extend around and enclose the inflation indicator 37 to protect it from damage by chewing.

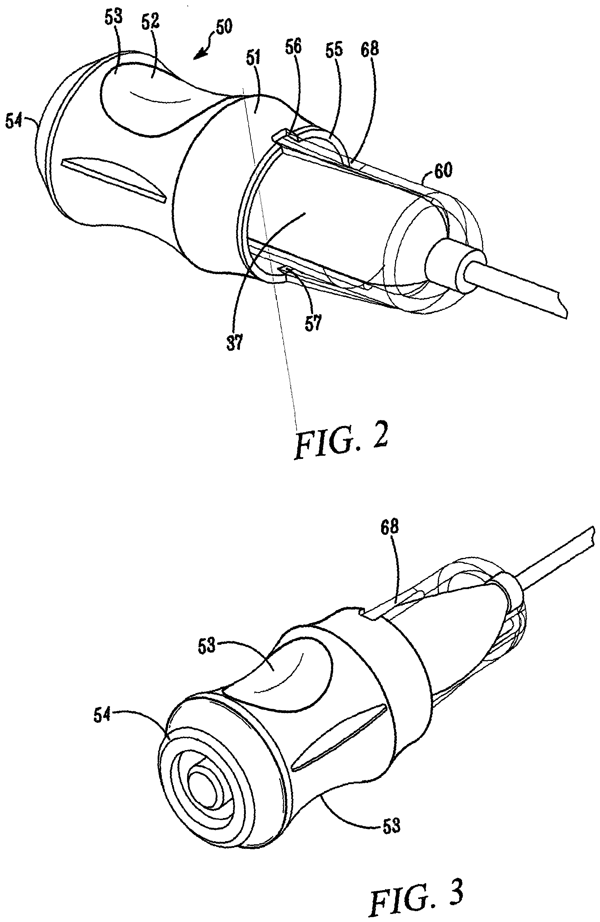

Figures 2 to 4 show the inflation indicator 37 before and after assembly of the protector enclosure 50 onto the inflation indicator 37. The enclosure 50 consists of two main parts. The first, machine-end part or base 51 is a moulding of a semi-rigid, opaque plastics material, such as having a Shore D hardness of about 50. The base 51 has a circular section with a waisted central region 52 formed with flats 53 around its circumference to improve the grip on the enclosure 50 between finger and thumb. The machine end 54 of the base 51 is open so that the machine end 40 of the inflation indicator 37 is accessible for inflation or deflation. At its opposite, patient end 55 the outer surface of the base 51 is formed with two opposite locking formations or notches 56 and 57.

The protector enclosure 50 is completed by a second part 60 in the form of a transparent cylindrical sleeve. The sleeve 60 is moulded of a stiff, transparent plastics material and is circular in section with an open machine end 62 having an outwardly projecting annular rim 63 the external diameter of which is such that it snaps into an internal retaining groove (not shown) around the inside of the base 51. The patient end 64 of the sleeve 60 is closed apart from a small opening 65 just large enough to receive the inflation line 31. The sleeve 60 also has two diametrically opposite slots 66 and 67 extending longitudinally of the sleeve. One slot 66 opens at its machine end into the open machine end 62 of the sleeve 60 and opens at its opposite end into the small patient-end opening 65 of the sleeve. The other slot 67 is shorter and terminates before the machine end opening 62 and the patient end opening 65. The longer slot 66 enables the sleeve 60 to be slid onto the inflation line 31 sideways and also gives the sleeve some resilience so that it can be squeezed to reduce the diameter at its machine end 62 and enable this to be inserted into the patient end of the base 51. The sleeve 60 also has two locking features 68 and 69 on opposite sides of its outer surface, midway between the two slots 66 and 67 and about half way along the length of the sleeve. The locking features 68 and 69 are shaped to clip onto the two notches 56 and 57 on outside of the base 51. Engagement of the locking features 68 and 69 on the sleeve 60 with the notches 56 and 57 on the base 51 prevents the sleeve rotating relative to the base.

The protective enclosure 50 could be permanently fitted onto the inflation indicator 37 by applying a bonding agent to the machine end of the sleeve 60 so that it becomes securely and permanently bonded to the base part 51. Alternatively, the sleeve 61 need not be bonded to the base 51 so that the protector 50 can be removed from the inflation indicator 37 when necessary.

The protector enclosure 50 is fitted on the inflation indicator 37 by first sliding the inflation line 31 sideways through the long slot 66 of the sleeve 61 at a location spaced a short distance away from the inflation balloon. The sleeve 61 is then oriented so that the opposite slots 66 and 67 align with the edges of the inflation balloon 38. The sleeve 61 can then be threaded rearwardly along the inflation line 31 and along the outside of the inflation indicator 37 as far as it will go. The assembly of the inflation indicator 37 and sleeve 61 is then slid rearwardly into the patient end of the base 51, aligning the locking features 68 and 69 on the sleeve with the notches on the base. The sleeve 61 is squeezed at the same time to compress the slots 66 and 67 so as to enable the rim 63 to enter the base 51 and then open into the internal retaining groove when released. Instead of loading the sleeve 61 onto the inflation indicator 37 first it would be possible to insert the inflation indicator into the base first and then slide the sleeve over and along the inflation indicator into the base.

With the inflation indicator 37 contained within the protective enclosure 50 the balloon 38 can inflate and deflate in the usual manner and the state of inflation of the indicator can be viewed through the transparent sleeve 60. If the child patient should chew on the assembly, the inflation indicator 37 will not be damaged because it is protected by the protector 50.

The protector 50 can be readily fitted to a conventional inflation indicator of a tracheostomy tube without the need for any special tools. The protector could be provided separately of the tracheostomy tube and assembled on the inflation indicator by the user, if needed. The protector need not be fitted by a clinical specialist but can be fitted by a child's carer at home as needed.

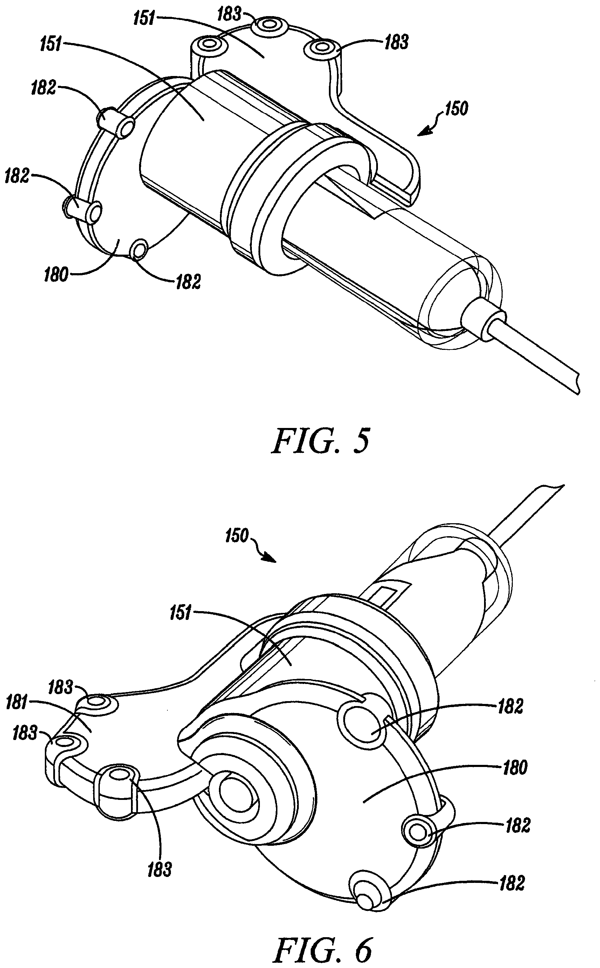

The protector could be provided with features specifically designed for the patient to chew so that he does not chew other parts of the protector. One example of a protector 150 with such features is shown in Figure 5 and 6 where the base 151 is integrally moulded with two projecting chew flanges 180 and 181. The flanges 180 and 181 are substantially flat and circular with three small protrusions 182 and 183 spaced around their edge. Both flanges 180 and 181 project outwardly of the base 151. One flange 180 has an axis normal to its plane extending parallel to the axis of the base 151. The other flange 181 has an axis normal to its plane extending orthogonally to the axis of the base 151. The patient can easily chew on these flanges 180 and 181 without causing any damage to the inflation indicator.

## Claims

1. A tracheostomy tube assembly including a tracheostomy tube (1) having a shaft (10) with an inflatable sealing cuff (13) towards its patient end (12) and an inflation line (31) communicating with the sealing cuff (13) and having an inflation indicator (37) towards its machine end, wherein the assembly also includes a protective enclosure (50, 150) extending around and enclosing the inflation indicator to protect it from damage by chewing, wherein the protective enclosure (50, 150) is transparent, at least in part, to enable the inflation indicator (37) to be viewed through the enclosure, wherein the protective enclosure (50, 150) has a first part (51) arranged to receive a machine end (40) of the inflation indicator (37) and a second part having a transparent sleeve (60) adapted to extend around the inflation indicator (37) and fit with the first part (51), wherein the second part (60) is open at its machine end (62) that fits with the first part (51), and wherein the second part (60) has an opening (65) at its patient end through which the inflation line (31) extends, and **characterised in that** the second part (60) has a slot (66) extending along its length between the open machine end (62) and the opening (65) at the patient end so that the inflation line (31) can be threaded through the slot (66).

2. A tracheostomy tube assembly according to Claim 1, **characterised in that** the second part (60) has a projecting rim (63) at one end adapted to engage a groove around the first part (51).

3. A tracheostomy tube assembly according to Claim 1 or 2, **characterised in that** the inflation indicator (37) has a substantially flat inflatable balloon (38), and that the protective enclosure (50) includes two opposite slots (66 and 67) aligned with opposite edges of the balloon (38).

4. A tracheostomy tube assembly according to Claim 1 or 2, **characterised in that** the protective enclosure (150) includes at least one projecting feature (180, 181) adapted to be chewed by a patient.

5. A tracheostomy tube assembly according to Claim 4, **characterised in that** the at least one projecting feature is provided by two flanges (180 and 181) oriented with the planes of the flanges substantially orthogonal to one another.

6. A method of forming a paediatric tracheostomy tube assembly including the steps of:
providing a paediatric tracheostomy tube (1) having a shaft (10) with an inflatable sealing cuff (13) towards its patient end (12) and an inflation line (31) communicating with the sealing cuff (13) and having an inflation indicator (37) towards its machine end; providing a protective enclosure (50, 150) in two parts comprising a first part (51) and a second part (60), wherein the enclosure is adapted to extend around and enclose the inflation indicator to protect it from damage by chewing in use, wherein the protective enclosure (50, 150) is transparent, at least in part, to enable the inflation indicator (37) to be viewed through the enclosure in use, wherein the first part is adapted to receive a machine end (40) of the inflation indicator (37) in use, wherein the second part has a transparent sleeve (60) adapted to extend around the inflation indicator (37) and fit with the first part (51) in use, wherein the second part (60) is open at its machine end (62) adapted to fit with the first part (51) in use, wherein the second part (60) has an opening (65) at its patient end through which the inflation line (31) extends in use and has a slot (66) extending along its length between the open machine end (62) and the opening (65) at the patient end so that the inflation line (31) can be threaded through the slot (66);
threading the inflation line (31) sideways through the slot (66); fitting the second part (60) over an inflatable part (38) of the inflation indicator (37), including sliding the second part (60) along the inflation line (31) to enclose the inflation indicator (37); and fitting the machine end of the second part (60) with the first part (51) such that the machine end (40) of the inflation indicator (37) is received in the first part (51).

## Patentansprüche

1. Tracheostomietubusanordnung mit einem Tracheostomietubus (1), der einen Schaft (10) mit einer aufblasbaren Dichtungsmanschette (13) an seinem Patientenende (12) und eine mit der Dichtungsmanschette (13) in Verbindung stehende Aufblasleitung (31) mit einem Aufblasindikator (37) an seinem Maschinenende aufweist, wobei die Anordnung auch eine Schutzhülle (50, 150) aufweist, die sich um den Aufblasindikator herum erstreckt und diesen umschließt, um ihn vor Beschädigung durch Kauen zu schützen, wobei die Schutzhülle (50, 150) zumindest teilweise transparent ist, damit der Aufblasindikator (37) durch die Hülle hindurch betrachtet werden kann, wobei die Schutzhülle (50, 150) ein erstes Teil (51), das zur Aufnahme eines Maschinenendes (40) des Aufblasindikators (37) ausgebildet ist, und ein zweites Teil mit einer transparenten Hülle (60) aufweist, die so ausgebildet ist, dass sie sich um den Aufblasindikator (37) herum erstreckt und mit dem ersten Teil (51) zusammenpasst, wobei das zweite Teil (60) an seinem Maschinenende (62), das mit dem ersten Teil (51) zusammenpasst, offen ist, und wobei das zweite Teil (60) an seinem Patientenende eine Öffnung (65) aufweist, durch die sich die Aufblasleitung (31) erstreckt, und **dadurch gekennzeichnet, dass** das zweite Teil (60) einen Schlitz (66) aufweist, der sich entlang seiner Länge zwischen dem offenen Maschinenende (62) und der Öffnung (65) am Patientenende erstreckt, so dass die Aufblasleitung (31) durch den Schlitz (66) gefädelt werden kann.

2. Tracheostomietubusanordnung nach Anspruch 1, **dadurch gekennzeichnet, dass** das zweite Teil (60) an einem Ende einen vorstehenden Rand (63) aufweist, der angepasst ist, in eine Nut um das erste Teil (51) herum einzugreifen.

3. Tracheostomietubusanordnung nach Anspruch 1 oder 2, **dadurch gekennzeichnet, dass** der Aufblasindikator (37) einen im Wesentlichen flachen aufblasbaren Ballon (38) aufweist und dass die Schutzhülle (50) zwei gegenüberliegende Schlitze (66 und 67) aufweist, die mit gegenüberliegenden Rändern des Ballons (38) ausgerichtet sind.

4. Tracheostomietubusanordnung nach Anspruch 1 oder 2, **dadurch gekennzeichnet, dass** die Schutzhülle (150) mindestens ein vorstehendes Element (180, 181) aufweist, das von einem Patienten gekaut werden kann.

5. Tracheostomietubusanordnung nach Anspruch 4, **dadurch gekennzeichnet, dass** das mindestens eine vorstehende Element durch zwei Flansche (180 und 181) gebildet ist, die so ausgerichtet sind, dass die Ebenen der Flansche im Wesentlichen orthogonal zueinander sind.

6. Verfahren zur Herstellung einer pädiatrischen Tracheostomietubusanordnung, umfassend die folgenden Schritte: Bereitstellen eines pädiatrischen Tracheostomietubus (1), der einen Schaft (10) mit einer aufblasbaren Dichtungsmanschette (13) an seinem Patientenende (12) und eine mit der Dichtungsmanschette (13) in Verbindung stehende Aufblasleitung (31) mit einem Aufblasindikator (37) an seinem Maschinenende aufweist; Bereitstellen einer zweiteiligen Schutzhülle (50, 150), die ein erstes Teil (51) und ein zweites Teil (60) umfasst, wobei die Hülle so angepasst ist, dass sie sich um den Aufblasindikator herum erstreckt und diesen umschließt, um ihn im Gebrauch vor Beschädigung durch Kauen zu schützen, wobei die Schutzhülle (50, 150) zumindest teilweise transparent ist, damit der Aufblasindikator (37) im Gebrauch durch die Umhüllung hindurch betrachtet werden kann, wobei das erste Teil so ausgebildet ist, dass es im Gebrauch ein Maschinenende (40) des Aufblasindikators (37) aufnimmt, wobei das zweite Teil eine durchsichtige Hülse (60) aufweist, die sich um den Aufblasindikator (37) herum erstreckt und im Gebrauch mit dem ersten Teil (51) zusammenpasst, wobei das zweite Teil (60) an seinem Maschinenende (62) offen ist und im Gebrauch mit dem ersten Teil (51) zusammenpasst, wobei das zweite Teil (60) an seinem Patientenende eine Öffnung (65) aufweist, durch die sich die Aufblasleitung (31) im Gebrauch erstreckt, und einen Schlitz (66) aufweist, der sich entlang seiner Länge zwischen dem offenen Maschinenende (62) und der Öffnung (65) am Patientenende erstreckt, so dass die Aufblasleitung (31) durch den Schlitz (66) gefädelt werden kann; Einfädeln der Aufblasleitung (31) seitwärts durch den Schlitz (66); Befestigen des zweiten Teils (60) über einem aufblasbaren Teil (38) des Aufblasindikators (37), einschließlich des Schiebens des zweiten Teils (60) entlang der Aufblasleitung (31), um den Aufblasindikator (37) zu umschließen; und Verbinden des Maschinenendes des zweiten Teils (60) mit dem ersten Teil (51), so dass das Maschinenende (40) des Aufblasindikators (37) im ersten Teil (51) aufgenommen wird.

## Revendications

1. Ensemble tube de trachéotomie comprenant un tube de trachéotomie (1) ayant une tige (10) avec un manchon d'étanchéité gonflable (13) vers son extrémité patient (12) et une ligne de gonflage (31) communiquant avec le manchon d'étanchéité (13) et ayant un indicateur de gonflage (37) vers son extrémité machine, dans lequel l'ensemble comprend également une enceinte de protection (50, 150) s'étendant autour et enfermant l'indicateur de gonflage pour le protéger des dommages causés par la mastication, dans lequel l'enceinte de protection (50, 150) est transparente, au moins en partie, pour permettre à l'indicateur de gonflage (37) d'être vu à travers l'enceinte, dans lequel l'enceinte de protection (50, 150) a une première partie (51) conçue pour recevoir une extrémité machine (40) de l'indicateur de gonflage (37) et une seconde partie (60) ayant un manchon transparent adapté pour s'étendre autour de l'indicateur de gonflage (37) et s'ajuster à la première partie (51),
dans lequel la seconde partie (60) est ouverte à son extrémité machine (62) qui s'adapte à la première partie (51), et dans laquelle la seconde partie (60) a une ouverture (65) à son extrémité patient à travers laquelle la ligne de gonflage (31) s'étend, et **caractérisé en ce que** la seconde partie (60) a une fente (66) s'étendant sur sa longueur entre l'extrémité machine ouverte (62) et l'ouverture (65) à l'extrémité patient de sorte que la ligne de gonflage (31) peut être enfilée à travers la fente (66).

2. Ensemble tube de trachéotomie selon la revendication 1, **caractérisé en ce que** la seconde partie (60) a un rebord saillant (63) à une extrémité adapté pour s'engager dans une rainure autour de la première partie (51).

3. Ensemble tube de trachéotomie selon la revendication 1 ou 2, **caractérisé en ce que** l'indicateur de gonflage (37) comporte un ballonnet gonflable (38) sensiblement plat, et que l'enceinte de protection (50) comporte deux fentes opposées (66 et 67) alignées sur les bords opposés du ballonnet (38).

4. Ensemble tube de trachéotomie selon la revendication 1 ou 2, **caractérisé en ce que** l'enceinte de protection (150) comprend au moins un élément en saillie (180, 181) adapté pour être mâché par un patient.

5. Ensemble tube de trachéotomie selon la revendication 4, **caractérisé en ce que** ledit au moins un élément en saillie est constitué de deux rebords (180 et 181) orientés avec les plans des rebords sensiblement orthogonaux l'un par rapport à l'autre.

6. Procédé de formation d'un ensemble tube de trachéotomie pédiatrique comprenant les étapes consistant à :
fournir un tube de trachéotomie pédiatrique (1) comportant une tige (10) avec un manchon d'étanchéité gonflable (13) vers son extrémité patient (12) et une ligne de gonflage (31) communiquant avec le manchon d'étanchéité (13) et comportant un indicateur de gonflage (37) vers son extrémité machine ;
fournir une enceinte de protection (50, 150) en deux parties comprenant une première partie (51) et une seconde partie (60), dans lequel l'enceinte est adaptée pour s'étendre autour et enfermer l'indicateur de gonflage pour le protéger des dommages causés par la mastication lors de l'utilisation, dans lequel l'enceinte de protection (50, 150) est transparente, au moins en partie, pour permettre à l'indicateur de gonflage (37) d'être vu à travers l'enceinte en cours d'utilisation, dans lequel la première partie est adaptée pour recevoir une extrémité machine (40) de l'indicateur de gonflage (37) en cours d'utilisation, dans lequel la seconde partie (60) comporte un manchon transparent adapté pour s'étendre autour de l'indicateur de gonflage (37) et s'adapter à la première partie (51) en cours d'utilisation, dans lequel la seconde partie (60) est ouverte à son extrémité machine (62) adaptée pour s'adapter à la première partie (51) en cours d'utilisation, dans lequel la seconde partie (60) a une ouverture (65) à son extrémité patient à travers laquelle la ligne de gonflage (31) s'étend en cours d'utilisation et comporte une fente (66) s'étendant sur toute sa longueur entre l'extrémité machine ouverte (62) et l'ouverture (65) à l'extrémité patient de sorte que la ligne de gonflage (31) puisse être enfilée à travers la fente (66) ;
enfiler la ligne de gonflage (31) latéralement à travers la fente (66) ; installer la seconde partie (60) sur une partie gonflable (38) de l'indicateur de gonflage (37), comprenant le coulissement de la seconde partie (60) le long de la ligne de gonflage (31) pour enfermer l'indicateur de gonflage (37) ; et ajuster l'extrémité machine de la seconde partie (60) avec la première partie (51) de telle sorte que l'extrémité machine (40) de l'indicateur de gonflage (37) soit reçue dans la première partie (51).
